# EUROPEAN PATENT SPECIFICATION

(11) **EP 1 812 422 B1**
(45) Date of publication and mention of the grant of the patent: **25.12.2013**
(21) Application number: 05802257.5
(22) Date of filing: 09.11.2005
(51) Int. Cl.: C07D 403/10, A61K 31/415

(54) **POLYMORPH FORM OF IRBESARTAN**
POLYMORPHE IRBESARTANFORM
FORME POLYMORPHE D'IRBESARTAN

(30) Priority: 11.11.2004 SI 200400308
(43) Date of publication of application: 01.08.2007
(73) Proprietor: LEK Pharmaceuticals d.d., 1526 Ljubljana (SI)
(72) Inventor: ANTONCIC, Ljubomir, 1000 Ljubljana (SI)
(74) Representative: Prüfer & Partner GbR European Patent Attorneys
(86) International application number: PCT/EP2005/011982
(87) International publication number: WO 2006/050923

(56) References cited:
- EP-A- 0 708 103
- EP-A- 1 089 994
- WO-A-03/050110
- WO-A-2004/062557
- WO-A-2004/089938
- WO-A-2005/051943
- WO-A-2006/001026
- VEESLER, S. ET AL.: "Phase Transitions in Supersaturated Drug Solution" ORGANIC PROCESS RESEARCH & DEVELOPMENT, vol. 7, 2003, pages 983-989, XP002376533
- BOUTONNET-FAGEGALTIER, N. ET AL.: "Molecular Mobility Study" JOURNAL OF PHARMACEUTICAL SCIENCES, vol. 91, 2002, pages 1548-1560, XP002376534
- BAUER, M. ET AL.: "NMR study of desmotropy in Irbesartan" J. CHEM. SOC. PERKIN TRANS. 2, 1998, pages 475-481, XP002376535
- GARCIA, E. ET AL.: "Dissolution and phase transition of pharmaceutical compounds" JOURNAL OF CRYSTAL GROWTH, 2002, pages 2233-2239, XP002376536

## Description

### FIELD OF THE INVENTION

The present invention relates a process for preparation of novel forms of irbesartan and pharmaceutical compositions containing it.

### BACKGROUND OF THE INVENTION

Irbesartan is an antihypertensive agent known from EP 454511. From EP 708103, which discloses their X-ray spectra, two polymorphs are known where form A can be produced form a solvent system containing less than 10% of water, while Form B from a system with more than 10% of water. The specific morphological variant of form A can be prepared having properties as disclosed in EP 1089994. Additional form has been disclosed in WO 04089938. Amorphous irbesartan is known from WO 03050110. It is said that Irbesartan produced as taught in EP 454511 is a fluffy material with relatively low bulk and tap densities and undesirable flow characteristics, which consequently has unadvantageous electrostatic properties, among them a high chargeability as measured by tribugeneration between -30 and -40 nanocoulomb/g (10⁻⁹As/g). Alternatively irbesartan could be prepared by complex process using sonifications and/or temperature oscillations according to EP 1089994 to exhibit a chargeability as measured by tribugeneration between -0 and -10 nanocoulomb/g.

According to EP 454511 a solid composition in form of tablets is prepared by mixing the active ingredient with a vehicle such as gelatine, starch, lactose, magnesium stearate, talc, gum Arabic or the like and can be optionally coated. The compositions containing from 20% to 70% by weight of irbesartan are known from EP 747050.

Irbesartan is administered in a unit dose up to 1 g, usually as the tablets containing from 75 mg up to 300 mg or 600 mg of the active ingredient. For the patient's compliance and comfort it is desirable to prepare tablets which are small and thus easy to swallow, however must have also suitable characteristics which are satisfactorily hardness, friability, disintegration needed for purpose of storage, handling and drug release characteristics.

To date, an irbesartan comprising pharmaceutical composition, such as tablet, which would incorporate a simply prepared active substance, and in which the proportion of the active substance would be as high as possible to give a relatively small easy swallowable tablet has not yet been realized.

### BRIEF DESCRIPTION OF THE DRAWINGS

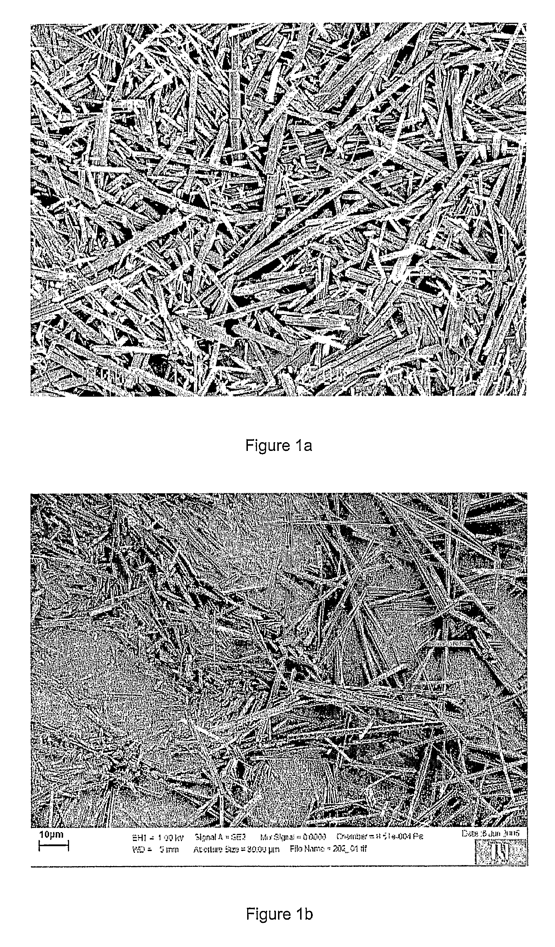

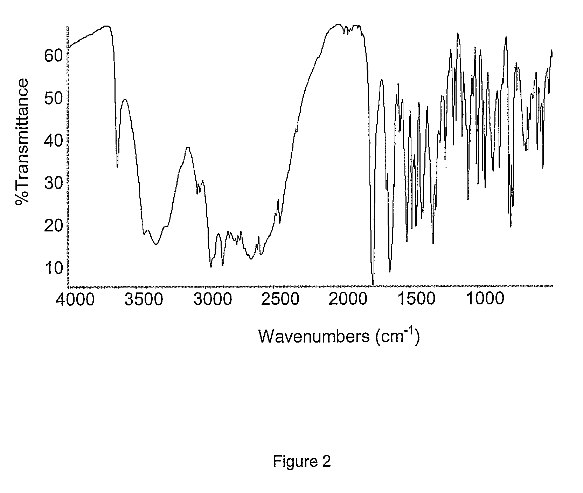

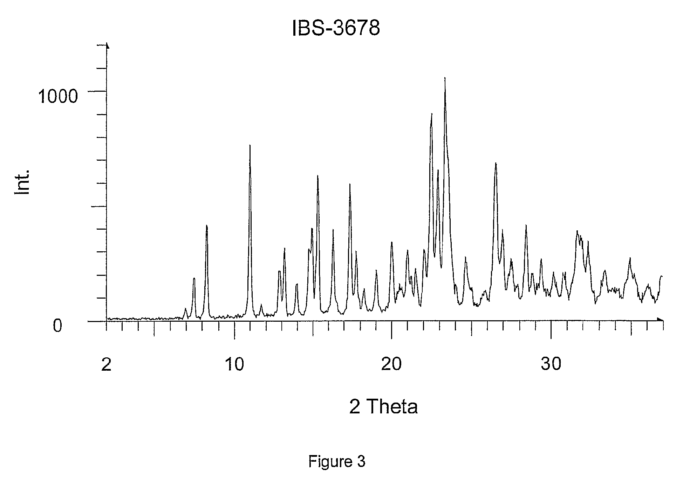

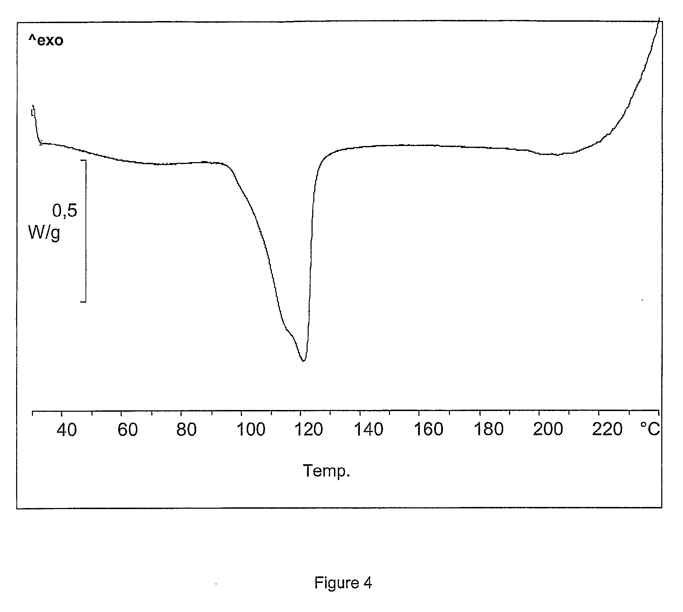

Figures 1 a and 1 bare SEM photographs of two batches of the new form of irbesartan

### DISCLOSURE OF THE INVENTION

The aspect of the invention is a process for preparing irbesartan which to 2-n-butyl-4-spirocyclopentane-1-[((2'-tetrazol-5-yl)biphenyl-4-yl)methyl]-2-imidazolin-5-one characterized by the following steps:
a) providing a clear solution of irbesartan in a solvent selected from the group consisting of C1 to C4 aliphatic alcohol, THF and acetone, preferably ethanol or propanol, at the temperature above 45 °C;
b) subsequently cooling the solution to the temperature bellow 35 °C in a manner that at 40 °C the solution is clear; and
c) subsequently isolating the formed crystals of irbesartan.

Preferred embodiments are set forth in claims 2 to 18.

New crystalline form of irbesartan can be used as a medicament.

It may be used for the manufacture of a medicament for treatment of hypertension. In a specific embodiment, the process further comprises a process characterized by steps:
a) granulation with water a first granulate consisting of mixture of irbesartan, optionally a second active ingredient, one or more binders, a first portion of one or more diluents, and one or more disintegrants;
b) addition thereto an antiadherent, a second portion of one or more diluents and an lubricant and tableting; and
c) (optionally) coating the manufucatured tablets.

Preferably the amounts of above ingredients relative to the weight of the composition will be: irbesartan above 70%; the optional second active ingredient, which is preferably hydrochlorotiazide in amount from 3 to 6%; one or more diluents in first portion ifrom 2,5 to 7,5%, and in second portion from 1 to 8%; one or more binders from 4 to 12%; one or more disintegrants is from 2 to 6%; and one or more antiadherents and lubricants from 1 to 2%.

### DETAILED DESCRIPTION OF THE INVENTION

We have experimented with irbesartan form A formulating it by conventional methods by combining it with excipients such as gelatine, starch, lactose, magnesium stearate, talc, gum Arabic and realized that high loadings, that is the high proportion of the active substance versus total mass of the pharmaceutical composition are not possible.

Continuing our research we have surprisingly realized that active substance with relatively low bulk and/or tap densities and/or undesirable flow characteristics, (even or because of having a fluffy appearance), however exhibiting more favorable chargeability and/or crystal habit, can be formulated into pharmaceutical composition in high loadings above 50%, preferably above 70%, most preferably above 75%. The upper level of the loading depends on the excipients used and is believed to be up to 90%, preferably up to 85%. The new form of irbesartan, having appearance as seen by SEM of interconnected, interlaced or interwoven needles or lamellas or plates can be incorporated into a tablet even at higher loadings. The pharmaceutical composition may comprise above 70% , preferably above 75% and below 90% by weight of simply produced irbesartan which does not posses a high chargeability, where irbesartan has crystal habit such that the ratio between largest (length) and smallest dimension (width) of the crystals is above 5, preferably above 6, more preferably above 10, still more preferably above 11 and most preferably above 13.

Typical relatively low bulk densities are for example around 0,2, preferably 0,16 g/mL, while tap densities around 0,3, preferably 0,28 g/mL and undesirable flow characteristics are stickiness and adherence to surfaces, around in this context means ± 0,05 g/mL.

Forms of irbesartan in accordance with our invention will comprise the compound 2-n-butyl-4-spirocyclopentane-1-[((2'-tetrazol-5-yl)biphenyl-4-yl)methyl]-2-imidazolin-5-one or its complexes, addition salts and other forms which do not affect the bioavailability, such as 2-n-butyl-4-spirocyclopentane-1-[((2'-tetrazol-5-yl)biphenyl-4-yl)methyl]-2-imidazolin-5-one hydrochloride or hydrobromide.

### New forms of irbesartan

A new form of irbesartan can be prepared having a crystal habit such that the ratio between the length and the width of the crystals is above 5:1, preferably above 10:1 and having favorable chargeability properties. In preferred embodiment more than 50%, preferably more than 65%, more preferably above 95% of all particles will exhibit described crystal habit. The percentage preferably relates to number of particles. Preferably it would exhibit same X-ray spectra as the substance prepared in EP 454511 and EP 1089994 (Form A as known from EP 708103). However the favorable chargeability properties means that substance in accordance with present invention does not posses a high electrostatic nature which would in one embodiment mean possessing a chargeability as measured by tribogeneration between -10 and -40 nanocoulomb/g, preferably between -10 and -30, nanocoulomb/g, more preferably between around 15 and 27 nanocoulombs/g .

One can prepare the new form of irbesartan from a hot clear solution of irbesartan. Hot solution will have temperature above 40 °C, preferably above 45 °C, more preferably around 60 °C, most preferably the temperature of reflux of solvents. If the solution is so slowly cooled down so that the solution is still clear at around 40 °C, the new form crystallizes. The process is surprising in that the crystals of new form will form even if the solution is agitated. The agitation can be for example sporadic shaking or stirring, for example with a suitable blade shaped stirrer at up to 40 rpm. After the solution has been cooled to 40 °C and it is still clear, the crystallization can be accelerated with seeding. The cooling can be continued below 40 °C, preferably bellow 35 °C, more preferably to room temperature (r. t.).

Preferably irbesartan should be completely dissolved in suitable solvent, preferably a C₁ to C₄ aliphatic alcohol, ether containing 2 to 6 C atoms, such as THF or C₃ to C₄ ketone, most preferably in ethanol, i-propanol, THF or acetone. As log as the complete dissolution is achieved, the concentration may vary for example from 1 g in 5 mL to 100 mL, preferably in alcohols from 1 g in 10 to 25 mL. The essential part of the invention is a slow rate of cooling of the clear solution, in one embodiment not exceeding 20 °C, preferably 10 °C per hour, more preferably even slower and in other embodiment taking from the temperature of reflux to the r.t. up to 10 hours, preferably 2 to 3 hours.

The yield and/or favorable physical properties (those allowing easy incorporation into a pharmaceutical composition) can be further improved in another embodiment of the invention, where small amount of the crystal prepared in a separate experiment as above are used as a seed for crystallization of irbesartan from a solution.

The new forms of irbesartan can be produced in substantially pure form or in a mixture with any other form. Depending on the desired characteristic, for example desired dissolution properties, the substantially pure polymorph forms can be incorporated into a pharmaceutical composition in pure form or alternatively a mixture thereof.

The described new forms of irbesartan, alone or in combination with another active, e.g. diuretic, preferably hydrochlorotiazide, have a potent antihypertensive activity and incorporated into a pharmaceutical composition can be in a form suitable for peroral or parental application. Pharmaceutical composition in accordance with this invention can be embodied for example in form of tablet, capsules, pellets, granules and supozitories or their combined forms. Solid pharmaceutical compositions (dosage forms) can be shielded, for example coated with the aim of increasing peletibility or regulating the disintegration or absorption.

The solid dosage forms comprising new forms of irbesartan can be prepared by conventional method. The favorable electrostatic properties and/or crystal habit allow the formation of a tablet which is convenient to the patient. Since irbesartan is administered in dosage up to 600 mg per tablet, this means that only small amount of excipients (constituting a pharmaceutically acceptable carrier) may be used. Tablet can be for example manufactured by direct compression though wet granulation is another commonly used technique. In wet granulation at least one of the ingredients can be mixed or contacted with liquid and further processed to provide aggregates, the liquid can be partially or completely removed and optionally other or more of the same ingredients may be further added and solid dosage forms manufactured.

Tableting compositions may have in addition to active pharmaceutical ingredient few or many components, depending upon the tableting method used, the release rate desired and other factors. For example, compositions of the present invention may contain inactive ingredients (excipients) which function as such as different fillers, binders, disintegrants, glidants, lubricants, antiadherents and excipients that enhance the absorption of drugs from gastrointestinal tract.

Suitable fillers or diluents may be selected from microcrystalline cellulose, powdered cellulose, lactose, starch, pregelatinized starch, sucrose, glucose, mannitol, sorbitol, calcium phosphate, calcium hydrogen phosphate, aluminium silicate, sodium chloride, potassium chloride, calcium carbonate, calcium sulphate, dextrates, dextrin, maltodextrin, glycerol palmitostearate, hydrogenated vegetable oil, kaolin, magenesium carbonate, magnesium oxide, polymethacrylates, talc, and others. Preferred fillers are microcrystalline cellulose and lactose, most preferable (optionally silicified) microcrystalline cellulose and lactose monohydrate. Suitable binders may be starch, pregelatinized starch, gelatine, sodium carboxymethylcellulose, polyvinylpyrrolidone, alginic acid, sodium alginate, acacia, carbomer, dextrin, ehylcellulose, guar gum, hydrogenated vegetable oil, methylcellulose, hydroxyethylcellulose, hydroxypropylcellulose, hydroxypropylmethylcellulose, glucose syrup, magnesium aluminium silicate, maltodextrin, polymethacrylates, zein. Preferably hydroxypropyl cellulose, hydroxypropyl methylcellulose and polyvinylpyrrolidone, most preferably polyvinylpyrrolidone are used. Suitable disintegrants may be selected from starch, pregelatinized starch, sodium starch glycolate, sodium carboxymethylcellulose, cross-linked sodium carboxymrethylcellulose, calcium carboxymethylcellulose, methylcellulose, microcrystalline cellulose, powdered cellulose, polacrilin potassium, cross-linked polivinylpyrrolidone, alginic acid, sodium alginate, colloidal silicon dioxide, guar gum, magnesium aluminium silicate, and others. Preferred disintegrants are sodium starch glycolate, cross-linked carboxymethylcellulose sodium, croscarmellose sodium and cross-linked polyvinylpyrrolidone, most preferably croscarmellose sodium. Suitable glidants may be magnesium stearate, calcium stearate, aluminium stearate, stearic acid, palmitic acid, cetanol, stearol, polyethylene glycols of different molecular weights, magnesium trisilicate, calcium phosphate, colloidal silicon dioxide, talc, powdered cellulose, starch and others. Preferred antiadherent is colloidal silicilon dioxide. Suitable lubricants may be selected from stearic acid, calcium, magnesium, zinc or aluminium stearate, siliconized talc, glycerol monostearate, glycerol palmitostearate, hydrogenated castor oil, hydrogenated vegetable oil, mineral oil, light mineral oil, polyethylene glycol, sodium benzoate, sodium lauryl sulphate, sodium stearyl fumarate, talc and others. Preferred lubricants are calcium or magnesium stearate and stearic acid, most preferable is sodium stearyl fumarate. Suitable absorption enhancers may be selected from surface active agents, fatty acids, middle chain glycerides, steroide detergents (salts of bile salts), acyl carnitine and alcanoloil choline (esters of carnitine and choline and fatty acids with middle chain and long chain), N-acyl derivatrives of alpha-amino acids and N-acyl derivatives of non-alpha-amino acids, chitosanes and other mucoadhesive polymers. Especially suitable absorption enhancers are sodium deoxycholate, sodium taurocholate, polisorbate 80, sodium lauryl sulfate, sodium dodecylsulfate, octanoic acid, sodium docusate, sodium laurate, glyceride monolaurate, stearic acid, palmitinic acid, palmitooleinic acid, glycerilmonooleate, sodium taurocholate, ethylenediaminetetraacetic acid, sodium edentate, sodium citrate, b-cyclodextrine and sodium salicylate.

Specifically the composition may comprise a second active ingredient, preferably a hydrochlorotiazide and a pharmaceutically acceptable carrier, which may comprise one or more fillers or diluents, binders, disintegrants, glidants or antiadherents, lubricants and excipients that enhance the absorption of drugs.

In additional embodiment the above binder is povidone; each diluent is selected form the group consisting of lactose monohydrate, microcrystalline cellulose and silicified microcrystalline cellulose; disintegrant is croscarmellose sodium; antiadherent is coloidal silicium dioxide; lubricant is sodium stearyl fumarate.

In accordance with our invention one can mix irbesartan with relatively small amount of excipients selected as described bellow, preferably so that ratio of irbesartan to excipients is above 5 to 1, preferably above 6 to 1 and subsequently granulate the mixture with suitable granulating liquid. Any granulating liquid can be selected based on solubilities of irbesartan and excipinets, however water a preferred. Surprisingly the granulate after drying does not any more exhibit undesirable flow characteristics and can be optionally with addition of a small amount of any additional excipients formulated into tablets, onto which a coating can be applied. For the film coat standard excipients are used that are most suitable for water film-coating formulation such as hypromellose and hydroxypropylcellulose as film formers, polethyleneglycol as plasticizer, lactose as soluble filler, titanium dioxide as white coloring agent and talc as anti-sticking agent. Other coloring agent such as ferric oxide can be added.

In the preferred embodiment the tablets containing 300 mg of irbesartan will not weight more than 400 mg, and can be prepared by granulating with water first granulate consisting_of mixture of irbesartan, optionally a second active ingredient, such as hydrochlorotiazide in amount about 3 to 6%; a binder, preferably in amount from 2,5 to 7,5%, more preferably about 5%; one or more diluents, preferably in amount from 4 to 12%, more preferably about 8%, a disintegrant, preferably in amount from 2 to 6%, more preferably about 4%. To said first granulate extragranular excipients: antiadherent in amount about 1%; one or more diluents in amount from about 1 to 8% diluent and an lubricant in amount of about 2% are added and mixture tableted. Analogously a tablet containing 150 mg of irbesartan will not weight more than 200 mg. All amounts are weight percents relative to the mass of the tablet and the term about means an amount ± 20 % , preferably ±10 % relative to the amount. Preferably the coating will not be more than about 1 to 2 % by weight to the composition.

### EXPERIMENTAL PART

Infrared spectra was obtained with Nicolet Nexus FTIR spectrophotometer. Samples were analyzed in KBr and scanned from 400 to 4000 cm⁻¹ with 16 scans and 2 cm⁻¹ resolution. Thermogram was obtained with Mettler Toledo DSC822^{e} differential scanning calorimeter. The sample was placed in an unsealed aluminium pan with one hole and heated at 5 °C /min in the temperature range from 30 °C to 240 °C in the nitrogen (100 mL/min).

Powder X-ray diffraction spectra of the sample was recorded on Philips PW1710 with reflexion technique: CuKα radiation, range from 2° to 37° 2Theta, step 0.04° 2Theta, integration time 1 sec.

From an X-ray diffraction pattern of a powdery substance one can establish differences among different crystal lattices, and can obtain information on level of order i.e. crystallinity where lover crystallinity causes peaks to broaden. The diffraction values for a crystalline substance will be substantially independent of the difractometer used, if the difractometer is calibrated the values can differ for about 0,05° 2 Theta, taking into account the rounding the differences in values lay in the order of ± 0,1° 2Theta, however the different recording conditions or differences in preparing or handling samples can cause the variations from the values reported for as much as ± 0,2 2Theta. The intensities of each specific diffraction peak are a function of various factors, one of those being a particle size and preferred orientation. Skilled person will recognize the polymorph form from the comparison od whole X-ray powder diffraction patterns and specifically from the strongest peaks or any two to five or more distinct peaks selected from the listed peaks.

A method of measuring electrostatic nature is a tribogeneration where relative charge on the powdered sample is measured with an electrometer equipped with a Faraday cup (NanoCoulumb Meter, Model 284, Monroe Electronics, USA). Prior to measurement, the charge is generated by strongly vibrating the sample (approximately 3 g) on a dry Petri dish (D= 24 cm) for 2 minutes. The charged sample is then transferred to a Faraday cup of the electrometer with a plastic spoon and its charge is measured by an electrometer. After the charge measurement the sample is weighted and the relative charge calculated. All measurements are performed at controlled dry atmosphere conditions (15% relative humidity and 23 °C). The substances in accordance with present invention preferably exhibits values between around - 27 in certain embodiments and around - 15 nanocoulomb/g in other embodiments, compared to values -30 to -40 nanocoulomb/g and 0 to -10 nanocoulomb/g for previously known forms

The chargeability so measured is - 26.5 ± 6·10⁻⁹ As/g for the sample of the irbesartan in accordance with our invention corresponding to Figure 1 a (1649) and 45.8 ± 4,9·10⁻⁹As/g in Figure 1b (3913D) with magnification 500x.

The shape of crystals is determined by an analysis of an image, taken by the scanning electron microscope (SEM), JEOL JXA840A at magnification 500-2000X. The length/width ratio of the crystal habit is determined by measuring length and width of a representative sample (N=200) of crystals on the image, using the image analyzing software Olympus DP-Soft, v- 3.2. The length/width ratio is calculated independently for each crystal. Finally, the number average of the ratio is determined and optionally histogram calculated.

The following table shows the properties of different batches of irbesartan:

| | Solvent from which the substance is crystalized | a (µm) | b (µm) | a : b | a : b conf. int.95% | chargeab. 10⁻⁹As/g |
|---|---|---|---|---|---|---|
| 1 | n-propanol | 14.9 ± 10.1 | 1.2 ± 1.4 | 13 ± 10 | 11.25 - 15.15 | - 33.7 ± 15 |
| 2 | l-propanol | 78 ± 68 | 6.7 ± 2.8 | 13.4 ± 1.9 | 11.1 - 15.7 | - 32 ± 4.6 |
| 3 | acetone | 20.7 ± 17.4 | 1.0 ± 0.5 | 23.3 ± 23 | 18.6 - 27.8 | - 45.8 ± 4.9 |
| 4 | methanol | 130 ± 96 | 7.9 ± 3.4 | 17 ± 12.9 | 14.6 - 20.4 | - 27.5 ± 5.6 |
| 5 | abs. ethanol | 58 ± 50 | 6.4 ± 1.2 | 9.5 ± 8 | 8.1 - 11.1 | - 17.1 ± 2.9 |
| 6 | abs. ethanol * | 32 ± 32 | 1.4 ± 0.6 | 22 ± 18 | 15.8 - 27.4 | - 27.9 ± 2.8 |
| 7 | 96% ethanol * | 21 ± 17 | 2.6±1.0 | 13 ± 9 | 10.1 - 15.9 | - 26.5 ± 6 |

| | | | | | | |
|---|---|---|---|---|---|---|
| * experiment with seeding | | | | | | |

Other batches, especially crystallized from THF and acetone had a : b ratios for example 4 ± 3; 6.1 ± 3.7 and changeability - 14.2 ± 2; - 15.6 ± 2.3·10⁻⁹As/g respectively.

In the preferred embodiment our invention is a process where 15 g of irbesartan is completely dissolved in 300 mL of absolute ethanol heated to temperature of reflux to provide a clear solution which is subsequently cooled during 2 to 3 hours to reach room temperature. While the solution is cooled, it is stirred all the time at 40 rpm. The rate of cooling and agitation is preferably such that while passing the temperature of 40 °C the solution is still clear. Bellow the temperature of 40 °C the crystals will begin to form and the rate of crystallization can optionally be accelerated by seeding. The formed crystals are separated by filtration or centrifugation and dried, optionally under vacuum and optionally at the elevated temperature, such as 55 °C.

In another preferred embodiment irbesartan hydrochloride is prepared by providing a clear solution of about 1 g of sodium salt of irbesartan in about 10 mL of water into which an excess of hydrochloric acid, preferably 7,7 mL or more of 1 N HCl and adjustment of pH to about 1 to 4, preferably to about pH around 2 and partial evaporation of a solvent to yield saturated solution and cooling one obtains crystalline irbesartan hydrochloride

Following examples further illustrate the invention, They are provided for illustrative purposes only and are not intended to limit in any way the invention.

### EXAMPLE 1

A stirred suspension of irbesartan (5 g) in n-propanol (100 mL) was heated at the temperature of reflux until clear solution was obtained. Solution was slowly cooled with periodically shaking (or slow stirring at 40 rpm) to a temperature of about 20°C in a period of 2-3 hours. Crystalline suspension was filtered and crystals were dried in vacuo at temperature of 60°C to obtain 3,6 g of product.

### EXAMPLE 2

A stirred suspension of irbesartan (5 g) in i-propanol (200 mL) was heated at the temperature of reflux until clear solution was obtained. Solution was filtered and slowly cooled with periodically shaking (or slow stirring at 40 rpm) to a temperature of about 20°C in a period of 2-3 hours. Crystalline suspension was filtered and crystals were dried in vacuo at a temperature of 60°C to obtain 4,16 g of product.

### EXAMPLE 3

A stirred suspension of irbesartan (5 g) in acetone (500 mL) was heated at the temperature of reflux until clear solution was obtained. Solution was filtered and slowly cooled with periodically shaking (or slow stirring at 40 rpm) to a temperature of about 20°C in a period of 2-3 hours. Crystalline suspension was filtered and crystals were dried in vacuo at a temperature of 60°C to obtain 2,16 g of product.

### EXAMPLE 4

A stirred suspension of irbesartan (5 g) in metanol (50 mL) was heated at the temperature of reflux until clear solution was obtained. Solution was slowly cooled with periodically shaking (or slow stirring at 40 rpm) to a temperature of about 20°C in a period of 2-3 hours. Crystalline suspension was filtered and crystals were dried in vacuo at a temperature of 60°C to obtain 4,2 g of product.

### EXAMPLE 5

A stirred suspension of irbesartan (5 g) in absolute ethanol (100 mL) was heated at the temperature of reflux until clear solution was obtained. Solution was slowly cooled with periodically shaking (or slow stirring at 40 rpm) to a temperature of about 20°C in a period of 2-3 hours. Crystalline suspension was filtered and crystals were dried in vacuo at a temperature of 60°C to obtain 4,11 g of product.

### EXAMPLE 6

A stirred suspension of irbesartan (15 g) in absolute ethanol (300 mL) was heated at the temperature of reflux until clear solution was obtained. Solution was slowly cooled with periodically shaking (or slow stirring at 40 rpm) to a temperature of about 20°C in a period of 2-3 hours. Meanwhile still clear solution was seeded at a temperature of about 40 °C. Crystalline suspension was filtered and crystals were dried in vacuo at a temperature of 55°C to obtain 12,28 g of product.

### EXAMPLE 7

A stirred suspension of irbesartan (15 g) in 96 % ethanol (300 mL) was heated at the temperature of reflux until clear solution was obtained. Solution was slowly cooled with periodically shaking (or slow stirring at 40 rpm) to a temperature of about 20°C in a period of 2-3 hours. Meanwhile still clear solution was seeded at a temperature of about 50 °C. Crystalline suspension was filtered and crystals were dried in vacuo at a temperature of 55°C to obtain 11,94 g of product.

### EXAMPLE 8

A stirred suspension of irbesartan (15 g) in n-propanol (174 mL) was heated at the temperature of reflux until clear solution was obtained. Solution was slowly cooled with periodically shaking (or slow stirring at 40 rpm) to a temperature of about 20°C in a period of 2-3 hours. Crystalline suspension was filtered and crystals were dried in vacuo at a temperature of 55°C to obtain 12,18 g of product.

### EXAMPLE 9

A stirred suspension of irbesartan (15 g) in i-propanol (330 mL) was heated at the temperature of reflux until clear solution was obtained. Solution was slowly cooled with periodically shaking (or slow stirring at 40 rpm) to a temperature of about 20°C in a period of 2-3 hours. Crystalline suspension was filtered and crystals were dried in vacuo at a temperature of 55°C to obtain 13,50 g of product.

### EXAMPLE 10

A stirred suspension of Irbesartan (15 g) in acetone (1050 mL) was heated at the temperature of reflux until clear solution was obtained. Solution was slowly cooled with periodically shaking (or slow stirring at 40 rpm) to a temperature of about 20°C in a period of 2-3 hours. Crystalline suspension was filtered and crystals were dried in vacuo at a temperature of 55°C to obtain 10,20 g of product.

### EXAMPLE 11

A stirred suspension of irbesartan (15 g) in tetrahydrofuran (255 mL) was heated at the temperature of reflux until clear solution was obtained. Solution was slowly cooled with periodically shaking (or slow stirring at 40 rpm) to a temperature of about 20°C in a period of 2-3 hours. Crystalline suspension was filtered and crystals were dried in vacuo at a temperature of 55°C to obtain 11,4 g of product.

### FORMULATION EXAMPLE 1

A tablet is prepared as follows: First granulate is prepared by mixing: irbesartan 300,00 mg and polyvinylpyrrolidone (K 25) 19,70 mg and lactose (70-100 mesh) 18,74 mg and microcrystalline cellulose (Avicel PH 101) 9,85 mg together with Na carboxy methyl cellulose (AcdiSol) 13,79 mg and granulating with water. Thereto silicon dioxide (Aerosil 200) 2,36 mg, sSilicified microcrystalline cellulose (Prosolv HD90) 21,67 mg and Na stearil fumarate (Pruv) 7,88 mg are added and tableted.

### FORMULATION EXAMPLE 2

A tablet is prepared by making first granulate as above which contains additionally 2,50 mg hydrochlorotiazide Thereto silicon dioxide (Aerosil 200) 2,36 mg, silicifed microcrystalline cellulose (Prosolv HD90) 9,17 mg and Na stearil fumarate (Pruv) 7,88 mg are added and tableted.

### FORMULATION EXAMPLE 3

A tablet is prepared by granulating with water first granulate consisting of irbesartan 150,00 mg, hydrochlorotiazide 12,50 mg and polyvinylpyrrolidone (K 25) 9,85 mg and lactose (70-100 mesh) 3,12 mg and microcrystalline cellulose (Avicel PH 101) 4,92 mg together with Na carboxy methyl cellulose (AcdiSol) 6,90 mg. Thereto silicon dioxide (Aerosil 200) 1,18 mg, silicified microcrystalline cellulose (Prosolv HD90) 4.58 mg and Na stearil fumarate (Pruv) 3,94 mg are added and tableted.

Although the present formulations contains high percentage of active substance alone or in combination with another active, rapid and complete drug release is achieved.

Accelerated stability studies show that the formulation exhibits good stability in the packaging that protects product from moisture.

## Claims

1. A process for preparing irbesartan which is 2-n-butyl-4-spirocyclopentane-1-[((2'-tetrazol-5-yl)biphenyl-4-yl)methyl]-2-imidazolin-5-one **characterized by** the following steps:
a) providing a clear solution of irbesartan in a solvent selected from the group consisting of C1 to C4 aliphatic alcohol, THF and acetone at the temperature above 45 °C;
b) subsequently cooling the solution to the temperature below 35 °C in a manner that at 40 °C the solution is clear;
c) subsequently isolating the formed crystals of irbesartan.

2. The process according to previous claim where in step a) irbesartan is completely dissolved in said solvent.

3. The process according to previous claim where the solution of step b) is slowly cooled at rate not exceeding 10 °C per hour.

4. The process according to previous two claims where the solution of step a) is seeded.

5. The process according to previous claim where the concentration of a clear solution is from 1 g of irbesartan in 5 mL to 100 mL of solvent.

6. The process according to previous claim where the concentration of a clear solution is from 1 g of irbesartan in 10 mL to 25 mL of solvent, where the solvent is selected from ethanol or propanol or mixture thereof.

7. The process according to any one of claims 1 to 6 where the solution is during cooling in step b) agitated by stirring where the stirring does not exceed 40 rpm.

8. The process according to any one of claims 1 to 7 whereupon the isolated crystals of irbesartan exhibit X-ray spectra of Form A.

9. The process according to any one of claims 1 to 8 whereupon the isolated crystals of irbesartan have chargeability as measured by tribogeneration between -15 and - 27 nanocoulombs/g.

10. The process according to any one of claims 1 to 9 whereupon the isolated crystals of irbesartan have crystal habit such that the average ratio between the largest and the smallest dimension of the crystals is above 10:1.

11. The process according to any one of claims 2 to 10 whereupon the isolated crystals of irbesartan have crystal habit such that the average ratio between the largest and the smallest dimension of the crystals is above 11:1.

12. The process according to any one of claims 1 to 11 whereupon the isolated crystals of irbesartan have appearance as seen by SEM of interconnected, interlaced or interwoven needles.

13. The process according to previous claim whereupon the isolated crystals of irbesartan have appearance as seen by SEM as on Figure 1.

14. The process according to any one of claims 1 to 11 whereupon the isolated crystals of irbesartan have bulk density around 0,2 g/mL.

15. The process according to any one of claims 1 to 13 whereupon the isolated crystals of irbesartan have tap density around 0,3 g/mL.

16. The process according to any one of claims 1 to 15, further comprising preparing a pharmaceutical composition comprising a crystalline form irbesartan.

17. The process according to claim 16, wherein the amount of irbesartan in the pharmaceutical composition is above 75% by weight.

18. The process according to any one of claims 16 or 17, wherein preparing the pharmaceutical composition is **characterized by** steps:
a) granulating with water a first granulate consisting of mixture of crystalline form of irbesartan, as defined in any of the claims 16 to 21, optionally a second active ingredient, one or more binders, a first portion of one or more diluents, and one or more disintegrants;
b) adding there to an antiadherent, a second portion of one or more diluents and an lubricant and tableting;
c) optionally coating the manufactured tablets.

## Patentansprüche

1. Verfahren zur Herstellung von Irbesartan, welches 2-n-Butyl-4-spirocyclopentan-1-[((2'-tetrazol-5-yl)biphenyl-4-yl)methyl]-2-imidazolin-5-on ist, **gekennzeichnet durch** die folgenden Schritte:
a) Bereitstellen einer klaren Lösung von Irbesartan in einem Lösungsmittel ausgewählt aus der Gruppe bestehend aus C1 bis C4 alipatischem Alkohol, THF und Aceton bei der Temperatur oberhalb von 45°C;
b) darauffolgend Kühlen der Lösung auf die Temperatur unterhalb von 35°C in einer Weise, dass bei 40°C die Lösung klar ist;
c) darauffolgend Isolieren der gebildeten Kristalle von Irbesartan.

2. Verfahren gemäß dem vorangehenden Anspruch, wobei in Schritt a) Irbesartan vollständig in besagtem Lösungsmittel gelöst wird.

3. Verfahren gemäß dem vorangehenden Anspruch, wobei die Lösung von Schritt b) langsam gekühlt wird bei einer Geschwindigkeitsrate, die 10°C pro Stunde nicht übersteigt.

4. Verfahren gemäß den beiden vorangehenden Ansprüchen, wobei die Lösung von Schritt a) geimpft wird.

5. Verfahren gemäß dem vorangehenden Anspruch, wobei die Konzentration von einer klaren Lösung von 1 g Irbesartan in 5 ml bis 100 ml Lösungsmittel beträgt.

6. Verfahren gemäß dem vorangehenden Anspruch, wobei die Konzentration von einer klaren Lösung von 1 g Irbesartan in 10 ml bis 25 ml Lösungsmittel beträgt, wobei das Lösungsmittel ausgewählt ist aus Ethanol oder Propanol oder einer Mischung davon.

7. Verfahren gemäß irgendeinem der Ansprüche 1 bis 6, wobei die Lösung während des Kühlens in Schritt b) durch Rühren agitiert wird, wobei das Rühren 40 Umdrehungen pro Minute nicht übersteigt.

8. Verfahren gemäß irgendeinem der Ansprüche 1 bis 7, wobei die isolierten Kristalle von Irbesartan Röntgenspektren von Form A aufweisen.

9. Verfahren gemäß irgendeinem der Ansprüche 1 bis 8, wobei die isolierten Kristalle von Irbesartan eine durch Reibungsaufladung gemessene Ladefähigkeit zwischen -15 und -27 Nanocoulomb/g aufweisen.

10. Verfahren gemäß irgendeinem der Ansprüche 1 bis 9, wobei die isolierten Kristalle von Irbesartan einen Kristallhabitus aufweisen, so dass das durchschnittliche Verhältnis zwischen der größten und der kleinsten Dimension der Kristalle oberhalb von 10:1 liegt.

11. Verfahren gemäß irgendeinem der Ansprüche 2 bis 10, wobei die isolierten Kristalle von Irbesartan einen Kristallhabitus aufweisen, so dass das durchschnittliche Verhältnis zwischen der größten und der kleinsten Dimension der Kristalle oberhalb von 11:1 liegt.

12. Verfahren gemäß irgendeinem der Ansprüche 1 bis 11, wobei die isolierten Kristalle von Irbesartan ein wie durch REM gesehenes Erscheinungsbild von miteinander verbundenen, ineinander verschlungenen oder miteinander verwobenen Nadeln aufweisen.

13. Verfahren gemäß dem vorangehenden Anspruch, wobei die isolierten Kristalle von Irbesartan ein wie durch REM gesehenes Erscheinungsbild wie in Figur 1 aufweisen.

14. Verfahren gemäß irgendeinem der Ansprüche 1 bis 11, wobei die isolierten Kristalle von Irbesartan eine Schüttdichte von ungefähr 0,2 g/ml aufweisen.

15. Verfahren gemäß irgendeinem der Ansprüche 1 bis 13, wobei die isolierten Kristalle von Irbesartan eine Klopfdichte von ungefähr 0,3 g/ml aufweisen.

16. Verfahren gemäß irgendeinem der Ansprüche 1 bis 15, ferner umfassend die Herstellung einer pharmazeutischen Zusammensetzung, welche eine kristalline Form von Irbesartan umfasst.

17. Verfahren gemäß Anspruch 16, wobei die Menge von Irbesartan in der pharmazeutischen Zusammensetzung oberhalb von 75 Gew.-% liegt.

18. Verfahren gemäß irgendeinem der Ansprüche 16 oder 17, wobei das Herstellen der pharmazeutischen Zusammensetzung **gekennzeichnet ist durch** die Schritte:
a) Granulieren mit Wasser eines ersten Granulats bestehend aus einer Mischung aus kristalliner Form von Irbesartan, wie in irgendeinem der Ansprüche 16 bis 21 definiert, wahlweise einem zweiten Wirkstoff, einem oder mehreren Bindemitteln, einem ersten Teil eines oder mehrerer Verdünnungsmittel und einem oder mehreren Sprengmitteln;
b) dazu Hinzufügen eines Antihaftmittels, eines zweiten Teils von einem oder mehreren Verdünnungsmitteln und eines Gleitmittels und Tablettieren;
c) wahlweise Beschichten der hergestellten Tabletten.

## Revendications

1. Procédé de préparation d'irbésartan qui est du 2-n-butyle-4-spirocyclopentane-1-[((2'-tétrazol-5-yl)biphényl-4-yl)méthyle]-2-imidazoli n-5-one, **caractérisé par** les étapes suivantes :
a) de préparation d'une solution transparente d'irbésartan dans un solvant sélectionné à partir du groupe constitué de l'alcool aliphatique en C1 à C4, du THF et de l'acétone à une température supérieure à 45° C ;
b) puis de refroidissement de la solution à une température inférieure à 35° C, de manière telle que la solution soit transparente à 40° C ;
c) puis d'isolation des cristaux d'irbésartan formés.

2. Procédé selon la revendication précédente, dans lequel, dans l'étape a), l'irbésartan est entièrement dissous dans ledit solvant.

3. Procédé selon la revendication précédente, dans lequel la solution de l'étape b) est refroidie lentement à une vitesse n'excédant pas 10° C par heure.

4. Procédé selon les deux revendications précédentes, dans lequel la solution de l'étape a) est ensemencée.

5. Procédé selon la revendication précédente, dans lequel la concentration de la solution transparente est de 1 g d'irbésartan dans 5 à 100 ml de solvant.

6. Procédé selon la revendication précédente, dans lequel la concentration de la solution transparente est de 1 g d'irbésartan dans 10 ml à 25 ml de solvant, dans lequel le solvant est sélectionné à partir d'éthanol ou de propanol ou d'un mélange de ceux-ci.

7. Procédé selon l'une quelconque des revendications 1 à 6, dans lequel la solution est, au cours du refroidissement de l'étape b), agitée par mise en rotation dans lequel la rotation n'excède pas 40 t/mn.

8. Procédé selon l'une quelconque des revendications 1 à 7, après lequel les cristaux d'irbésartan isolés présentent des spectres de rayons X de forme A.

9. Procédé selon l'une quelconque des revendications 1 à 8, après quoi les cristaux isolés d'irbésartan présentent une capacité de charge telle que mesurée par tribo-génération comprise entre -15 et -27 nanocoulombs/g.

10. Procédé selon l'une quelconque des revendications 1 à 9, après quoi les cristaux isolés d'irbésartan présentent une forme caractéristique de cristal de telle sorte que le rapport moyen entre la dimension la plus grande et la plus faible des cristaux est supérieure à 10:1.

11. Procédé selon l'une quelconque des revendications 2 à 10, après lequel les cristaux isolés d'irbésartan présentent une forme caractéristique de cristal, de telle sorte que le rapport moyen entre la dimension la plus grande et la plus faible des cristaux est supérieur à 11:1.

12. Procédé selon l'une quelconque des revendications 1 à 11, après lequel les cristaux isolés d'irbésartan présentent une apparence, telle que vue par SEM, d'aiguilles interconnectées, entrelacées ou entretissées.

13. Procédé selon la revendication précédente, après quoi les cristaux isolés d'irbésartan présentent une apparence, telle que vue par SEM, similaire à la figure 1.

14. Procédé selon l'une quelconque des revendications 1 à 11, après lequel les cristaux isolés d'irbésartan présentent une densité brute de 0,2 g/ml environ.

15. Procédé selon l'une quelconque des revendications 1 à 13, après lequel les cristaux isolés d'irbésartan présentent une densité tassée de 0,3 g/ml environ.

16. Procédé selon l'une quelconque des revendications 1 à 15, comprenant en outre la préparation d'une composition pharmaceutique comprenant de l'irbésartan de forme cristalline.

17. Procédé selon la revendication 16, dans lequel la quantité d'irbésartan dans la composition pharmaceutique est supérieure à 75 % en poids.

18. Procédé selon l'une quelconque des revendications 16 ou 17, dans lequel la préparation de la composition pharmaceutique est **caractérisée par** les étapes :
a) de granulation à l'eau d'un premier granulat constitué par un mélange de forme cristalline d'irbésartan, tel que défini selon l'une quelconque des revendications 16 à 21, en variante, d'un second ingrédient actif, d'un ou plusieurs liants, d'une première partie d'un ou plusieurs diluants, et d'un ou plusieurs agents de désagrégation ;
b) d'ajout à celui-ci d'un agent anti-adhérent, d'une seconde partie d'un ou plusieurs diluants et d'un lubrifiant et de mise sous forme de comprimés ;
c) en variante, d'enrobage des comprimés fabriqués.
